# EUROPÄISCHE PATENTSCHRIFT

(11) **EP 1 642 558 B1**
(45) Veröffentlichungstag und Bekanntmachung des Hinweises auf die Patenterteilung: **03.10.2007**
(21) Anmeldenummer: 05104405.5
(22) Anmeldetag: 24.05.2005
(51) Int. Cl.: A61F 15/00

(54) **Pflasterspender**
Dispenser for dressings
Distributeur de condiments

(30) Priorität: 30.09.2004 DE 102004047607
(43) Veröffentlichungstag der Anmeldung: 05.04.2006
(73) Patentinhaber: wero-medical Werner Michallik GmbH & Co. KG, 65232 Taunusstein (DE)
(72) Erfinder: Michallik, Wolfram, 65187, Wiesbaden (DE)
(74) Vertreter: Weber, Roland

(56) Entgegenhaltungen:
- DE-A1- 19 736 990
- US-A- 4 194 624
- US-A- 4 549 653

## Beschreibung

Die vorliegende Erfindung betrifft einen Pflasterspender mit darin vorgesehenen Pflastern sowie eine Wandhalterbox mit darin untergebrachten Pflasterspendern.

Heftpflaster zum Verbinden kleinerer Verletzungen, wie Schnitt- oder Schürfwunden, gehören zur Grundausstattung beim Erste-Hilfe-Material im Haushalt wie auch in Gewerbebetrieben. Handelsüblich werden Pflaster in unterschiedlichen Mengen und Größen in Pappschachteln oder Papier- oder Kunststoffhüllen angeboten. Aus hygienischen Gründen sind die Pflaster darin üblicherweise noch einmal einzeln abgepackt. Da das Auspacken eines einzelnen Pflasters im Verletzungsfall zeitaufwendig und für den Verletzten mit beispielsweise einer Handverletzung schwierig zu handhaben ist, sind Pflasterspender erhältlich, aus denen sich einzelne Pflaster mit einer Hand mühelos entnehmen lassen. Besonders in Gewerbebetrieben sind solche Pflasterspender häufig als Wandhalterboxen in der Nähe des Arbeitsplatzes für einen schnellen Zugriff montiert. Bekannte Wandhalterboxen bestehen üblicherweise aus Kunststoff und sind mit einem Verschlußdeckel versehen, um das Eindringen von Schmutz zu verhindern. In solche Wandhalterboxen werden austauschbare Pflasterspender eingesetzt, welche die Pflaster für eine einzelne Entnahme bereitstellen.

Einzelpflaster bestehen üblicherweise aus einer Rückenlage, de auf einer Seite eine Haftmittelschicht und eine Wundauflage aufweist. Um insbesondere die Wundauflage vor Verschmutzung und die mit Haftmittel versehenen Bereiche der Rückenlage vor einem Verkleben bei der Lagerung zu schützen, sind über der Haftmittelschicht und der Wundauflage üblicherweise eine oder mehrere Deck- oder Schutzfolien vorgesehen, die vor der Benutzung des Pflasters zum Abdecken einer Wunde abzuziehen sind.

Fingerverbände sind Pflaster, die bezüglich Form und Größe der Rückenlage sowie Anordnung und Größe der Wundauflage speziell für das Verbinden von kleineren Verletzungen an den Fingern der Hand ausgestaltet sind. Fingerverbände besitzen üblicherweise eine längliche Rückenlage in den Standardgrößen 12 x 2 cm, 12 x 3 cm und 18 x 2 cm sowie eine Wundauflage, die, bezogen auf die Längserstreckung der Rückenlage, von der Mitte der Rückenlage so weit versetzt ist, daß sich von der Wundauflage aus ein kurzes Rückenlagenstück mit Haftmittel zum Ansetzen des Pflasters an einem Finger neben der Wunde und ein deutlich längeres Rückenlagenstück mit Haftmittel zum mehrfachen Umwickeln des Fingers erstrecken.

Die DE-OS 28 49 680 beschreibt einen Vorratsbehälter für eine als Pflasterspender ausgebildete, mehrere Heftpflaster aufweisende Heftpflasterpackung, wobei der Vorratsbehälter und die Heftpflasterpackung so ausgebildet sind, daß die in den Vorratsbehälter eingesetzte Heftpflasterpackung nur mit Hilfe eines speziellen Werkzeugs einfach aus dem Vorratsbehälter zu entnehmen ist. Die Heftpflasterpackung weist hintereinander mehrere Reihen von Doppellagen aus Papier mit dazwischen ausgebildeten Taschen und in den Taschen untergebrachten Pflastern auf. Die Pflaster ragen aus oberen Öffnungen der Taschen heraus und werden so für eine einzelne Entnahme bereitgestellt. Die Heftpflasterpackung der DE-OS 28 49 680 für herkömmliche kurze Pflaster ausgelegt und eignet sich nicht für längere Pflaster, wie Fingerverbände, da diese sehr weit und ungeordnet aus den oberen Öffnungen der Taschen herausragen würden. Zudem hat diese Heftpflasterpackung den Nachteil, daß beim Herausnehmen eines Pflasters auch die Seite der Rückenlage ergriffen werden muß, die nicht mit einer Deck- oder Schutzfolie bedeckt ist. Dies führt dazu, daß das Pflaster bereits bei der Entnahme verschmutzt wird, wenn der Benutzer, üblicherweise der Verletzte, das Pflaster mit schmutzigen oder blutigen Fingern greift, was bei Verletzungen im Arbeitsbereich häufig der Fall sein wird.

Für alle Arten von Pflastern, aber insbesondere für Fingerverbände, wäre es wünschenswert, ein Spendersystem bereitzustellen, das es einem Verletzten erlaubt, ein Pflaster mit einer Hand aus dem Spender zu entnehmen und das Pflaster ohne Zuhilfenahme der anderen Hand auf eine Wunde aufzubringen. Darüber hinaus wäre es wünschenswert, daß auch stark länglich ausgebildete Pflaster, insbesondere Fingerverbände, möglichst kompakt und platzsparend sowie weitestgehend vor Verschmutzung geschützt in dem Spendersystem bereitgestellt werden, da diese in der betrieblichen Erste Hilfe besonders häufig verwendet werden, es bislang jedoch an geeigneten Spendersystemen mangelt. Es ist daher eine Aufgabe der vorliegenden Erfindung, einen solchen Pflasterspender bereitzustellen.

Gelöst wird diese Aufgabe durch einen Pflasterspender mit darin vorgesehenen Pflastern, wobei
der Pflasterspender mehrere Lagen aus einem bogenförmigen Material umfaßt und jeweils zwei Lagen aus bogenförmigem Material derart zueinander angeordnet und abschnittsweise miteinander verbunden, vorzugsweise verklebt sind, daß dazwischen von wenigstens einer Seite zugängliche Taschen für die Aufnahme von Pflastern ausgebildet sind,
die Pflaster jeweils eine Rückenlage und auf einer Seite der Rückenlage eine Haftmittelschicht, eine Wundauflage und zwei die Haftmittelschicht und Wundauflage überdeckende Deckfolien aufweisen, wobei sich die Deckfolien von den jeweils gegenüberliegenden Enden der Rückenlage oder darüber hinaus ragend bis über wenigstens einen Abschnitt der Wundauflage erstrecken und die Enden der Deckfolien über der Wundauflage vorzugsweise einander überlappen,
die Pflaster an einer Falistelle so gefaltet sind, daß Abschnitte der nicht mit Haftmittelschicht, Wundauflage und Deckfolien versehenen Rückseite der Rückenlage aufeinander zu liegen kommen,
und auf der Innenseite der Taschen, die von zwei Lagen aus bogenförmigem Material gebildet sind, ein Haftmittel vorgesehen ist, an welchem eine der beiden Deckfolien des in der Tasche vorgesehenen Pflasters haftet.

Die in dem Pflasterspender ausgebildeten Taschen sind dafür ausgelegt, den größten Teil der Pflaster zu umschließen und somit vor Verschmutzung zu schützen. Darüber hinaus halten die Taschen die einzelnen Pflaster dem Benutzer für eine einfache Entnahme entgegen. Nur ein relativ kurzer Abschnitt der Pflaster steht aus den Taschen zum Ergreifen der Pflaster und Herausziehen aus dem Spender hervor.

Besonders vorteilhaft ist die Faltung der Pflaster in der Weise, daß die Abschnitte der nicht mit Haftmittelschicht, Wundauflage und Deckfolien versehenen Rückseite der Rückenlage aufeinander zu liegen kommen. Insbesondere bei langgestreckten Pflastern wird hierdurch Platz eingespart, und der gesamte Pflasterspender erhält eine kompakte und gut zu handhabende Höhe. Beim Herausnehmen eines Pflasters aus dem Spender ergreift der Benutzer nur die aufgrund der Faltung nach außen weisende Deckfolie, welche nach dem Abziehen von der Pflasterrückenlage verworfen wird. Beim Herausnehmen des Pflasters aus dem Spender bleibt das Pflaster daher sauber.

Erfindungsgemäß besonders vorteilhaft ist es, daß auf der Innenseite der Taschen, die von zwei Lagen aus bogenförmigem Material gebildet sind, ein Haftmittel vorgesehen ist, an welchem eine der beiden Deckfolien des in der Tasche vorgesehenen Pflasters haftet. Hierdurch wird eine der beiden Deckfolien des Pflasters beim Herausziehen des Pflasters aus dem Spender festgehalten und von der Rückenlage abgezogen. Die festgehaltene Deckfolie verbleibt in der Tasche des Pflasterspenders. Ohne daß der Benutzer erst noch eine Deckfolie abziehen muß, ist das Pflaster dadurch schon für das Aufbringen und Fixieren an der Haut im Bereich der Verletzung bereit. Der Benutzer bringt den freiliegenden und mit Haftmittel versehenen Abschnitt der Rückenlage neben der Wunde auf die Haut, so daß die Wundauflage über der Wunde zu liegen kommt. Mit einer zur Verfügung stehenden Hand zieht er dann die zweite Deckfolie ab und verklebt den verbleibenden Abschnitt der Rückenlage mit der Haut auf der gegenüberliegenden Seite der Wunde. Bei einem Fingerverband wird der längere freie Abschnitt der Rückenlage dann mehrfach um den verletzten Finger gewickelt, um eine gute Haftung des Pflasters zu gewährleisten.

Zweckmäßigerweise ist das Haftmittel an der Innenseite der Tasche als Klebstoffstreifen ausgebildet, der an der Innenseite der Tasche quer zur Längserstreckung des Pflasters verläuft. Hierdurch wird ein sicheres Haften der einen Deckfolie an der Innenseite der Tasche gewährleistet.

Bei einer bevorzugten Ausführungsform der Erfindung ist die Falistelle, an der das Pflaster gefaltet ist, bezogen auf die Längserstreckung der Rückenlage, von der Mitte der Rückenlage versetzt angeordnet, so daß das eine der beiden freien Enden der Rückenlage aufgrund der Faltung in einem Abstand von dem gegenüberliegenden freien Ende der Rückenlage zu liegen kommt. Dies hat sich insbesondere bei Fingerverbänden als besonders vorteilhaft erwiesen. Zweckmäßigerweise sollte die Falistelle nicht im Bereich der Wundauflage und nicht unmittelbar daran angrenzend vorgesehen sein, um die Wundauflage selbst nicht falten zu müssen und diese nicht zu beschädigen. Vorzugsweise beträgt der Abstand der beiden freien Enden der Rückenlage aufgrund bzw. nach der Faltung 1/12 bis 1/8 der Gesamtlänge der Rückenlage.

Weitere vorteilhafte Ausgestaltungen der vorliegenden Erfindung ergeben sich aus den anhängenden Patentansprüchen. Demnach ist es besonders zweckmäßig, insbesondere bei als Fingerverbänden verwendeten Pflastern, wenn die Wundauflage, bezogen auf die Längserstreckung der Rükkenlage, von der Mitte der Rückenlage versetzt angeordnet ist, wobei die Mitte der Wundauflage von der Mitte der Rückenlage, jeweils bezogen auf die Längserstreckung, um 1/6 bis 1/3, vorzugsweise 1 /3,5 bis 1 /3, besonders bevorzugt etwa 1/4 der Länge der Rückenlage versetzt angeordnet ist. Bei dieser Ausgestaltung sollte der von der Wundauflage aus kürzere Abschnitt der Rückenlage derjenige sein, von dem die Deckfolie beim Entnehmen des Pflasters aus dem Spender abgezogen wird und in der Tasche verbleibt. Dadurch lassen sich die oben genannten Vorteile beim Aufbringen des Pflasters auf eine Wunde gut verwirklichen.

Der erfindungsgemäße Pflasterspender kann nebeneinander zwischen zwei Lagen aus bogenförmigem Material genau eine Tasche für ein Pflaster aufweisen. Dies kann für breite Pflaster zweckmäßig sein. Vorteilhaft weist der erfindungsgemäße Pflasterspender jedoch nebeneinander mehrere Taschen, besonders bevorzugt drei Taschen, zwischen jeweils zwei Lagen aus bogenförmigem Material auf. Hierdurch kann in kompakter Form eine Vielzahl von Pflastern in dem Pflasterspender bereitgestellt werden.

Bei einer weiteren ganz besonders bevorzugten Ausführungsform des erfindungsgemäßen Pflasterspenders sind mehrere Doppellagen mit Taschen aus bogenförmigem Material an einer Seite, die den Taschenöffnungen gegenüberliegt, miteinander so verbunden, daß sich der Pflasterspender von der Stelle ausgehend, an der die Doppellagen miteinander verbunden sind, in Richtung der Taschenöffnungen fächerartig aufweitet. Dies gewährleistet eine kompakte und einfache Herstellung des erfindungsgemäßen Pflasterspenders und ein einfaches Einführen desselben in beispielsweise eine Wandhalterbox.

Wie oben bereits ausgeführt, ist es besonders vorteilhaft, wenn in jeder der zwischen den Lagen aus bogenförmigem Material ausgebildeten Taschen jeweils genau ein Pflaster vorgesehen ist. Selbstverständlich können die Lagen aus bogenförmigem Material in einer alternativen Ausführungsform auch so miteinander verbunden sein, daß sie weniger Taschen, wie beispielsweise nur eine Tasche, ausbilden und in jeder Tasche mehrere Pflaster untergebracht sind. Da die Pflaster innerhalb der Taschen durch die Anheftung der einen Deckfolie an der Innenseite der Taschen gehalten werden, besteht keine Gefahr, daß die Pflaster in den Taschen umkippen und von außen nicht mehr greifbar sind.

Besonders vorteilhaft ist es, insbesondere bei Pflastern, die als Fingerverband verwendet werden, wenn die Pflaster bzw. die Rückenlagen der Pflaster eine im wesentlichen rechteckige, längliche Form aufweisen, wobei das Verhältnis von Länge zu Breite 3:1 bis 3:9, vorzugsweise 4:1 bis 8:1, besonders bevorzugt 5:1 bis 7:1, ganz besonders bevorzugt etwa 6:1 beträgt. Eine für Fingerpflaster geeignete Pflasterlänge liegt im Bereich von 6 cm bis 16 cm, vorzugsweise von 8 cm bis 14 cm und besonders bevorzugt bei etwa 12 cm. Für den erfindungsgemäßen Pflasterspender geeignete Pflaster haben weiterhin zweckmäßigerweise eine Breite von 1 cm bis 5 cm, vorzugsweise eine Breite von 1,5 cm bis 3 cm, besonders bevorzugt eine Breite von etwa 2 cm. Zweckmäßig ist es auch, wenn die Wundauflagen der Pflaster im wesentlichen die gleiche Breite wie die Rückenlage aufweisen und eine Länge, die im wesentlichen 1/10 bis 1/3, vorzugsweise 1/6 bis 1/4 der Länge der Rükkenlage entspricht.

Das bogenförmige Material, aus dem der erfindungsgemäße Pflasterspender hergestellt ist, kann jedes vom Fachmann als geeignet angesehene Material sein. Preiswert und gut zu verarbeiten sind Papier, Pappe oder Kunststoffolie, wobei bogenförmiges Material aus Papier besonders bevorzugt ist.

Bei einer weiteren bevorzugten Ausführungsform des erfindungsgemäßen Pflasterspenders sind weiterhin zwei Materiallagen, vorzugsweise aus Papier, Pappe oder Kunststoff, vorgesehen, welche die mehreren fächerartig miteinander verbundenen Doppellagen aus bogenförmigem Material umschließen. Mit Umschließen ist hiermit gemeint, daß die zwei Materiallagen im wesentlichen die flächigen Vorder- und Rückseiten des Pflasterspenders bilden. Vorteilhaft ist es, wenn diese zwei Materiallagen aus einer etwas festeren Pappe als die Doppellagen bestehen, um die mit den Pflastern befüllten Doppellagen vor einer Beschädigung zu schützen.

Die Erfindung umfaßt weiterhin eine Wandhalterbox mit einem oder mehreren darin untergebrachten Pflasterspendern der vorbeschriebenen Art. Die Wandhalterbox weist zweckmäßigerweise wenigstens ein Fach für die Aufnahme von solchen Pflasterspendern auf, wobei das Fach im Inneren von der Fachöffnung zum Fachboden hin keilförmig verjüngend ausgebildet ist. Die fächerartig ausgebildete Ausführungsform des erfindungsgemäßen Pflasterspenders, bei der die Doppellagen mit Taschen auf einer Seite miteinander verbunden sind, paßt so optimal in das Fach der Wandhalterbox.

Die Erfindung wird nachfolgend anhand einer besonders bevorzugten Ausführungsform in Verbindung mit den anhängenden Figuren weiter erläutert.
- Figur 1: zeigt ein Pflaster des erfindungsgemäßen Pflasterspenders in der Draufsicht ohne Deckfolien.
- Figur 2: zeigt das Pflaster gemäß Figur 1 mit einer Deckfolie.
- Figur 3: zeigt das Pflaster der Figuren 1 und 2 mit zwei Deckfolien.
- Figur 4: zeigt die Anordnung eines Pflasters gemäß den Figuren 1 bis 3 in einer Tasche des erfindungsgemäßen Pflasterspenders im Querschnitt von der Seite.
- Figur 5: zeigt eine perspektivische schematische Darstellung des erfindungsgemäßen Pflasterspenders.

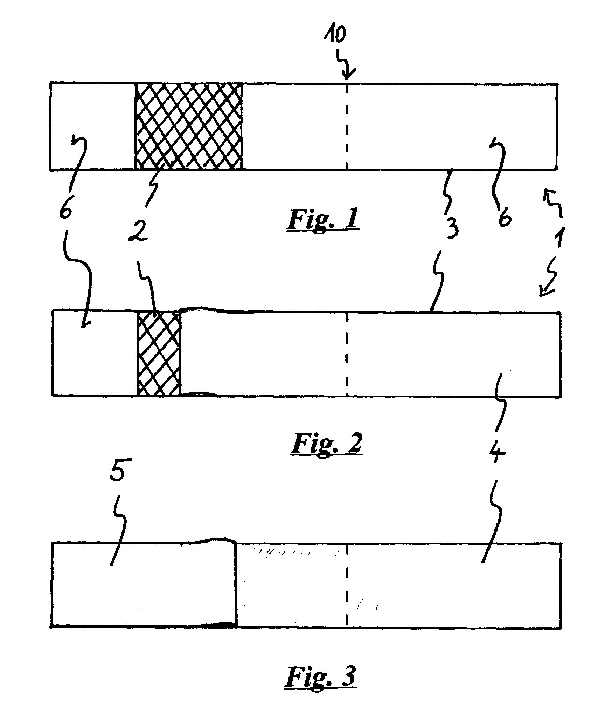

Die Figuren 1 bis 3 zeigen in der Draufsicht ein als Fingerverband ausgebildetes Pflaster 1 mit einer Rückenlage 3, einer darauf aufgebrachten Haftmittelschicht 6 und einer Wundauflage 2. Die Wundauflage 2 ist, bezogen auf die Längserstreckung der Rückenlage 3, von der Mitte der Rückenlage 3 versetzt angeordnet, so daß ein kürzerer und ein längerer Abschnitt der Rückenlage 3 mit zugänglicher Haftmittelschicht 6 verbleiben. Figuren 2 und 3 zeigen Deckfolien 4 und 5, wobei die Deckfolie 4 sich über den längeren freien Abschnitt der Rückenlage 3 erstreckt und die Deckfolie 5 über den kürzeren Abschnitt der Rückenlage 3. An den in Längsrichtung einander gegenüberliegenden Enden der Rückenlage 3 ragen die Deckfolien 4 und 5 jeweils ein kurzes Stück über die Enden hinaus. Beide Deckfolien 4 und 5 überlappen einander über der Wundauflage 2, wobei der Endabschnitt der kürzeren Deckfolie 5 über dem Endabschnitt der längeren Deckfolie 4 zu liegen kommt. Durch gestrichelte Linien ist in den Figuren 1 bis 3 die Faltstelle 10 angegeben, an der das Pflaster vor dem Einbringen in den Pflasterspender gefaltet wird.

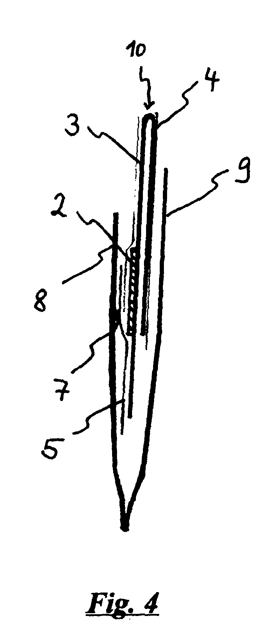

Figur 4 zeigt eine Tasche des erfindungsgemäßen Pflasterspenders im Querschnitt mit zwei Lagen 8 und 9 aus bogenförmigem Material, vorzugsweise Papier, und dem in der gebildeten Tasche angeordneten gefalteten Pflaster 1 gemäß Figur 3. Auf der Innenseite der Tasche ist an dem bogenförmigen Material 8 ein Klebstoffstreifen 7 vorgesehen, durch welchen die Deckfolie 5 mit der Innenseite des bogenförmigen Materials 8 fest verbunden ist. Beim Herausziehen des Pflasters 1 aus der Tasche des Pflasterspenders wird die Deckfolie 5 somit festgehalten, von dem Pflaster abgezogen und in der Tasche zurückgehalten. Üblicherweise faltet sich dabei der unterhalb des Klebstoffstreifens 7 befindliche Abschnitt der Deckfolie nach oben in Richtung der Taschenöffnung.

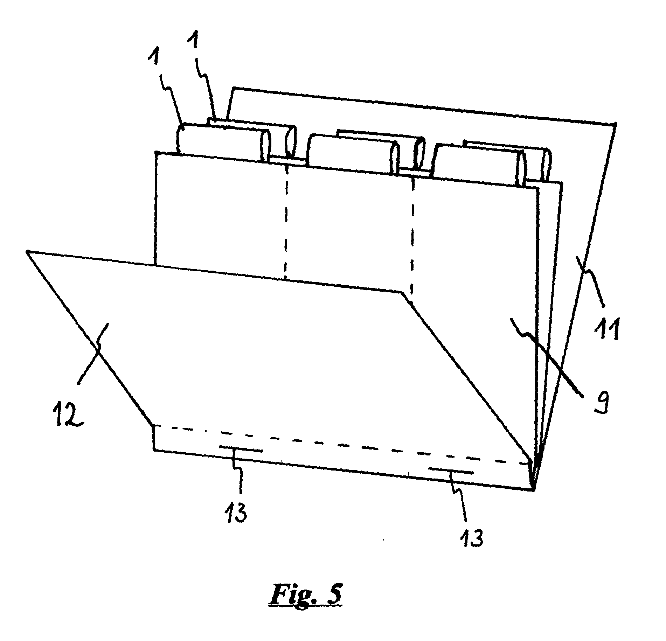

Figur 5 zeigt eine schematische Darstellung des erfindungsgemäßen Pflasterspenders, bei welchem jeweils zwei Lagen 8 und 9 aus bogenförmigem Material derart zueinander angeordnet und albschnittsweise miteinander verbunden sind, daß sie drei Taschen ausbilden. In jeder der drei Taschen ist ein Pflaster 1 in der Anordnung gemäß Figur 4 vorgesehen. Mehrere Doppellagen aus bogenförmigem Material mit darin enthaltenen Pflastern sind in der in Figur 5 dargestellten Ausführungsform des erfindungsgemäßen Pflasterspenders an der Unterseite miteinander verbunden, so daß der gesamte Pflasterspender nach oben hin fächerartig aufgeweitet ist. Vor und hinter den mehreren Doppellagen aus bogenförmigem Material mit Pflastern sind weiterhin zwei Materiallagen 11 und 12 zum Schutz vor Beschädigung vorgesehen. Die Verbindung zwischen den mehreren Doppellagen mit Pflastern und diesen zwei schützenden Materiallagen im unteren Abschnitt ist dadurch realisiert, daß die unteren Abschnitte der Doppellagen in einen gefalteten Pappbogen eingelegt und mit Heftklammern 13 zusammengeheftet sind.

## Patentansprüche

1. Pflasterspender mit darin vorgesehenen Pflastern, wobei
der Pflasterspender mehrere Lagen aus einem bogenförmigen Material umfaßt und jeweils zwei Lagen aus bogenförmigem Material derart zueinander angeordnet und abschnittsweise miteinander verbunden, vorzugsweise verklebt sind, daß dazwischen von wenigstens einer Seite zugängliche Taschen für die Aufnahme von Pflastern (1) ausgebildet sind,
die Pflaster (1) jeweils eine Rückenlage (3) und auf einer Seite der Rückenlage (3) eine Haftmittelschicht (6), eine Wundauflage (2) und zwei die Haftmittelschicht (6) und Wundauflage (3) überdeckende Deckfolien (4, 5) aufweisen, wobei sich die Deckfolien (4, 5) von den jeweils gegenüberliegenden Enden der Rückenlage (3) oder darüber hinaus ragend bis über wenigstens einen Abschnitt der Wundauflage (2) erstrecken und die Enden der Deckfolien (4, 5) über der Wundauflage (3) vorzugsweise einander überlappen,
die Pflaster (1) an einer Faltstelle (10) so gefaltet sind, daß Abschnitte der nicht mit Haftmittelschicht (6), Wundauflage (2) und Deckfolien (4, 5) versehenen Rückseite der Rückenlage (3) aufeinander zu liegen kommen,
und auf der Innenseite der Taschen, die von zwei Lagen aus bogenförmigem Material gebildet sind, ein Haftmittel (7) vorgesehen ist, an welchem eine der beiden Deckfolien (4, 5) des in der Tasche vorgesehenen Pflasters (1) haftet.

2. Pflasterspender nach Anspruch 1, **dadurch gekennzeichnet, daß** das Haftmittel (7) als Klebstoffstreifen ausgebildet ist, der an einer Innenseite der Tasche quer zur Längserstreckung des Pflasters (1) verläuft.

3. Pflasterspender nach einem der vorangegangenen Ansprüche, **dadurch gekennzeichnet, daß** die Faltstelle (10), bezogen auf die Längserstreckung der Rückenlage (3), von der Mitte der Rückenlage (3) versetzt angeordnet ist, so daß das eine der beiden freien Enden der Rückenlage (3) aufgrund der Faltung in einem Abstand von dem gegenüberliegenden freien Ende der Rückenlage (3) zu liegen kommt, vorzugsweise in einem Abstand der 1/12 bis 1/8 der Gesamtlänge der Rückenlage (3) beträgt.

4. Pflasterspender nach einem der vorangegangenen Ansprüche, **dadurch gekennzeichnet, daß** die Wundauflage (2), bezogen auf die Längserstreckung der Rückenlage (3), von der Mitte der Rückenlage (3) versetzt angeordnet ist, wobei die Mitte der Wundauflage (2) von der Mitte der Rückenlage (3), jeweils bezogen auf die Längserstreckung, um 1/6 bis 1/3, vorzugsweise 1/3,5 bis 1/5, besonders bevorzugt etwa 1/4 der Länge der Rückenlage (3) versetzt angeordnet ist.

5. Pflasterspender nach einem der vorangegangenen Ansprüche, **dadurch gekennzeichnet, daß** zwischen zwei Lagen aus bogenförmigem Material wenigstens eine Tasche, vorzugsweise mehrere Taschen, besonders bevorzugt genau drei Taschen ausgebildet sind.

6. Pflasterspender nach einem der vorangegangenen Ansprüche, **dadurch gekennzeichnet, daß** mehrere Doppellagen mit Taschen aus bogenförmigem Material an einer Seite, die den Taschenöffnungen gegenüberliegt, miteinander verbunden sind, so daß sich der Pflasterspender von der Stelle ausgehend, an der die Doppellagen miteinander verbunden sind, in Richtung der Taschenöffnungen fächerartig aufweitet.

7. Pflasterspender nach einem der vorangegangenen Ansprüche, **dadurch gekennzeichnet, daß** in jeder der zwischen den Lagen aus bogenförmigem Material ausgebildeten Taschen jeweils genau ein Pflaster vorgesehen ist.

8. Pflasterspender nach einem der vorangegangenen Ansprüche, **dadurch gekennzeichnet, daß** die Pflaster bzw. die Rückenlagen (3) der Pflaster eine im wesentlichen rechteckige, längliche Form, aufweisen, wobei das Verhältnis von Länge zu Breite 3:1 bis 9:1, vorzugsweise 4:1 bis 8:1, besonders bevorzugt 5:1 bis 7:1, ganz besonders bevorzugt etwa 6:1 beträgt.

9. Pflasterspender nach einem der vorangegangenen Ansprüche, **dadurch gekennzeichnet, daß** die Pflaster (1) bzw. die Rückenlagen (3) der Pflaster (1) eine Länge von 6 cm bis 16 cm, vorzugsweise eine Länge von 8 cm bis 14 cm, besonders bevorzugt eine Länge von etwa 12 cm, und eine Breite von 1 cm bis 5 cm, vorzugsweise eine Breite von 1,5 cm bis 3 cm, besonders bevorzugt eine Breite von etwa 2 cm aufweisen.

10. Pflasterspender nach einem der vorangegangenen Ansprüche, **dadurch gekennzeichnet, daß** die Wundauflagen (2) der Pflaster (1) im wesentlichen die gleiche Breite wie die Rükkenlage (3) aufweisen und eine Länge, die im wesentlichen 1/10 bis 1/3, vorzugsweise 1/6 bis 1/4 der Länge der Rückenlage (3) entspricht.

11. Pflasterspender nach einem der vorangegangenen Ansprüche, **dadurch gekennzeichnet, daß** das bogenförmige Material aus Papier, Pappe oder Kunststoffolie, vorzugsweise aus Papier besteht.

12. Pflasterspender nach einem der vorangegangenen Ansprüche, **dadurch gekennzeichnet, daß** die Pflaster (1) Fingerverbände sind.

13. Pflasterspender nach einem der vorangegangenen Ansprüche, **dadurch gekennzeichnet, daß** er weiterhin zwei Materiallagen, vorzugsweise aus Papier, Pappe oder Kunststoff, umfaßt, welche die mehreren fächerartig miteinander verbundenen Doppellagen aus bogenförmigem Material umschließen.

14. Wandhalterbox mit einem oder mehreren darin untergebrachten Pflasterspendern nach einem der vorangegangenen Ansprüche.

15. Wandhalterbox nach Anspruch 14, **dadurch gekennzeichnet, daß** die Wandhalterbox wenigstens ein Fach für die Aufnahme von Pflasterspendern nach einem der Ansprüche 1 bis 13 aufweist, wobei das Fach im Inneren von der Fachöffnung zum Fachboden hin keilförmig verjüngend ausgebildet ist.

## Claims

1. A plaster dispenser having plasters provided therein, wherein
the plaster dispenser includes a plurality of layers of an arcuate material and each two layers of arcuate material are arranged relative to each other and connected together in portion-wise manner, preferably by adhesive, in such a way that provided therebetween are pockets accessible from at least one side for accommodating plasters (1),
the plasters (1) each have a backing layer (3) and on one side of the backing layer (3) an adhesive layer (6), a wound contact layer (2) and two cover films (4, 5) which cover over the adhesive layer (6) and the wound contact layer (3), wherein the cover films (4, 5) extend from the respective opposite ends of the backing layer (3) or projecting therebeyond to over at least a portion of the wound contact layer (2) and the ends of the cover films (4, 5) preferably mutually overlap over the wound contact layer (3),
the plasters (1) are folded at a fold location (10) in such a way that portions of the rear side of the backing layer (3), which is not provided with adhesive layer (6), wound contact layer (2) and cover films (4, 5), come to lie upon each other,
and on the inside of the pockets which are formed by two layers of arcuate material there is provided an adhesive (7) to which one of the two cover films (4, 5) of the plaster (1) provided in the pocket adheres.

2. A plaster dispenser according to claim 1 **characterised in that** the adhesive (7) is in the form of an adhesive strip which extends at an inside of the pocket in transverse relationship with the longitudinal extent of the plaster (1).

3. A plaster dispenser according to one of the preceding claims **characterised in that** the fold location (10) is arranged displaced from the centre of the backing layer (3) with respect to the longitudinal extent of the backing layer (3) so that one of the two free ends of the backing layer (3) by virtue of the folding comes to lie at a spacing from the oppositely disposed free end of the backing layer (3), preferably at a spacing which is 1/12 to 1/8 of the total length of the backing layer (3).

4. A plaster dispenser according to one of the preceding claims **characterised in that** the wound contact layer (2) is arranged displaced from the centre of the backing layer (3) with respect to the longitudinal extent of the backing layer (3), wherein the centre of the wound contact layer (2) is arranged displaced from the centre of the backing layer (3), in each case with respect to the longitudinal extent, by from 1/6 to 1/3, preferably from 1/3.5 to 1/5, particularly preferably about 1/4, of the length of the backing layer (3).

5. A plaster dispenser according to one of the preceding claims **characterised in that** at least one pocket, preferably a plurality of pockets, particularly preferably precisely three pockets, are provided between two layers of arcuate material.

6. A plaster dispenser according to one of the preceding claims **characterised in that** a plurality of double layers with pockets of arcuate material are connected together at a side which is in opposite relationship to the pocket openings so that the plaster dispenser expands fan-like in the direction of the pocket openings from the location at which the double layers are connected together.

7. A plaster dispenser according to one of the preceding claims **characterised in that** precisely one respective plaster is provided in each of the pockets formed between the layers of arcuate material.

8. A plaster dispenser according to one of the preceding claims **characterised in that** the plasters or the backing layers (3) of the plasters are of a substantially rectangular, elongate shape, wherein the ratio of length to width is 3:1 to 9:1, preferably 4:1 to 8:1, particularly preferably 5:1 to 7:1, quite particularly preferably about 6:1.

9. A plaster dispenser according to one of the preceding claims **characterised in that** the plasters (1) or the backing layers (3) of the plasters (1) are of a length of 6 cm to 16 cm, preferably a length of 8 cm to 14 cm, particularly preferably a length of about 12 cm, and a width of 1 cm to 5 cm, preferably a width of 1.5 cm to 3 cm, particularly preferably a width of about 2 cm.

10. A plaster dispenser according to one of the preceding claims **characterised in that** the wound contact layers (2) of the plasters (1) are of substantially the same width as the backing layers (3) and are of a length which substantially corresponds to 1/10 to 1/3, preferably 1/6 to 1/4 of the length of the backing layer (3).

11. A plaster dispenser according to one of the preceding claims **characterised in that** the arcuate material comprises paper, cardboard or plastic material, preferably paper.

12. A plaster dispenser according to one of the preceding claims **characterised in that** the plasters (1) are finger bandages.

13. A plaster dispenser according to one of the preceding claims **characterised in that** it further includes two material layers, preferably of paper, cardboard or plastic material, which enclose the plurality of double layers of arcuate material, which are connected together fan-like.

14. A wall holder box comprising one or more plaster dispensers according to one of the preceding claims, which are disposed therein.

15. A wall holder box according to claim 14 **characterised in that** the wall holder box has at least one compartment for accommodating plaster dispensers according to one of claims 1 to 13, wherein the compartment is of a configuration which in the interior narrows in a wedge configuration from the compartment opening to the compartment bottom.

## Revendications

1. Distributeur de pansements dans lequel il est prévu des pansements,
le distributeur de pansements comportant plusieurs couches de matériau arqué et deux couches de matériau arqué étant disposées l'une par rapport à l'autre et reliées, avantageusement collées, par portions de façon à former entre elles des poches accessibles depuis au moins un côté et destinées à recevoir des pansements (1),
les pansements (1) comportant chacun une couche postérieure (3) et, d'un côté de la couche postérieure (3), une couche médiane adhésive (6), une couche côté blessure (2) et deux feuilles supérieures (4, 5) recouvrant la couches médiane adhésive (6) et la couche côté blessure (3), les feuilles supérieures (4, 5) s'étendant depuis les extrémités opposées de la couche postérieure (3) ou faisant saillie jusqu'au-dessus d'au moins une portion de la couche côté blessure (2) et les extrémités des feuilles supérieures (4, 5) au-dessus de la couche côté blessure (3) se recouvrant avantageusement,
les pansements (1) étant pliés au niveau d'une pliure (10) de telle sorte que des portions du côté arrière de la couche postérieure (3), qui n'est pas dotée de la couche médiane adhésive (6), de la couche côté blessure (2) et des feuilles supérieures (4, 5), viennent se placer l'une sur l'autre,
et du côté intérieur des poches, qui sont formées de deux couches de matériau arqué, un adhésif (7) étant prévu auquel adhère l'une des deux feuilles supérieures (4, 5) du pansement (1) prévu dans la poche.

2. Distributeur de pansements selon la revendication 1, **caractérisé en ce que** l'adhésif (7) est conformé en bande adhésive qui s'étend, sur un côté intérieur de la poche, transversalement à l'extension longitudinale du pansement (1).

3. Distributeur de pansements selon l'une des revendications précédentes, **caractérisé en ce que** la pliure (10) est placée en étant décalée du milieu de la couche postérieure (3), par référence à l'extension longitudinale de la couche postérieure (3), de sorte que l'une des deux extrémités libres de la couche postérieure (3) vient se placer, en raison de la pliure, à une certaine distance de l'extrémité libre opposée de la couche postérieure (3), avantageusement à une distance comprise entre 1/12 et 1/8 de la longueur totale de la couche postérieure (3).

4. Distributeur de pansements selon l'une des revendications précédentes, **caractérisé en ce que** la couche côté blessure (2) est disposée en étant décalée du milieu de la couche postérieure (3), par référence à l'extension longitudinale de la couche postérieure (3), le milieu de la couche côté blessure (2) étant disposé en étant décalé du milieu de la couche postérieure (3) d'une distance de 1/6 à 1/3, avantageusement de 1/3,5 à 1/5, de façon particulièrement préférée de 1/4 environ, de la longueur de la couche postérieure (3), à chaque fois par référence à l'extension longitudinale.

5. Distributeur de pansements selon l'une des revendications précédentes, **caractérisé en ce qu'**au moins une poche, avantageusement plusieurs poches, de façon particulièrement préférée exactement trois poches, sont conformées entre deux couches de matériau arqué.

6. Distributeur de pansements selon l'une des revendications précédentes, **caractérisé en ce que** plusieurs doubles couches, dotées de poches de matériau arqué, sont reliées sur un côté qui est opposé aux ouvertures des poches de sorte que le distributeur de pansements s'élargit en éventail en direction des ouvertures de poche en partant de l'endroit où les doubles couches sont reliées.

7. Distributeur de poche selon l'une des revendications précédentes, **caractérisé en ce qu'**un seul pansement est prévu dans chacune des poches conformées entre les couches de matériau arqué.

8. Distributeur de pansements selon l'une des revendications précédentes, **caractérisé en ce que** les pansements respectivement la couche postérieure (3) des pansements ont une forme allongée, sensiblement rectangulaire, le rapport de la longueur sur la largeur étant de 3:1 à 9:1, avantageusement de 4:1 à 8:1, de façon particulièrement préférée de 5:1 à 7:1, et de façon tout particulièrement préférée de 6:1 environ.

9. Distributeur de pansements selon l'une des revendications précédentes, **caractérisé en ce que** les pansements (1) respectivement la couche postérieure (3) des pansements (1) a une longueur de 6 cm à 16 cm, avantageusement une longueur de 8 cm à 14 cm, de façon particulièrement préférée une longueur d'environ 12 cm, et une largeur de 1 cm à 5 cm, avantageusement une largeur de 1,5 cm à 3 cm, de façon particulièrement préférée une largeur d'environ 2 cm.

10. Distributeur de pansements selon l'une des revendications précédentes, **caractérisé en ce que** la couche côté blessure (2) des pansements (1) a sensiblement la même largeur que la couche postérieure (3) et une longueur qui correspond sensiblement de 1/10 à 1/3, avantageusement de 1/6 à 1/4, fois la longueur de la couche postérieure (3).

11. Distributeur de pansements selon l'une des revendications précédentes, **caractérisé en ce que** le matériau arqué est du papier, du carton ou un film de matière plastique, avantageusement du papier.

12. Distributeur de pansements selon l'une des revendications précédents, **caractérisé en ce que** les pansements (1) sont des pansements pour doigts.

13. Distributeur de pansements selon l'une des revendications précédentes, **caractérisé en ce qu'**il comporte en outre deux couches de matériau, avantageusement de papier, de carton ou de matière plastique, qui entourent les doubles couches de matériau arqué, reliés en éventail.

14. Boîte murale dans laquelle sont montés un ou plusieurs distributeurs de pansements selon l'une des revendications précédentes.

15. Boîte murale selon la revendication 14, **caractérisé en ce que** la boîte murale comporte au moins un compartiment destiné à recevoir des distributeurs de pansements selon l'une des revendications 1 à 13, le compartiment étant conformé à l'intérieur de sorte qu'il s'amincit en forme de coin de l'ouverture du compartiment vers le fond du compartiment.
